# EUROPEAN PATENT APPLICATION

(11) **EP 1 162 458 A1**
(43) Date of publication of application: **12.12.2001**
(21) Application number: 00401567.3
(22) Date of filing: 05.06.2000
(51) Int. Cl.: G01N 33/50, G01N 33/92, C07K 14/705

(54) **Methods and compositions for modulating oxidized LDL transport**

(71) Applicant: INSTITUT NATIONAL DE LA SANTE ET DE LA RECHERCHE MEDICALE (INSERM), 75654 Paris Cédex 13 (FR)
(72) Inventor: McGregor, John, 69003 Lyon (FR); Malaud, Eric, 69006 Lyon (FR)
(74) Representative: Becker, Philippe

(57) **Abstract**

The present invention relates to the fields of biology and pharmacology. This invention relates, generally, to methods and compositions for modulating oxLDL activity or transport. The present invention resides more specifically in methods for screening compounds that modulate oxLDL activity, more particularly oxLDL cellular uptake. The present invention more particularly describes novel methods of selecting or identifying compounds that can modulate oxLDL cellular uptake by CD36 polypeptides in various cells, such as macrophages. The invention also relates to the use of such compounds, in particular for research, development, improvement, diagnosis, therapeutic or prophylactic treatment of various pathological conditions. The invention also relates to the products or compositions, such as CD36 variants, recombinant cells, vectors, kits, and the like, which can be used for implementing the above methods or for therapeutic purposes.

## Description

The present invention relates to the fields of biology and pharmacology. This invention relates, generally, to methods and compositions for modulating oxLDL activity or transport. The present invention resides more specifically in methods for screening compounds that modulate oxLDL activity, more particularly oxLDL cellular uptake. The present invention more particularly describes novel methods of selecting or identifying compounds that can modulate oxLDL cellular uptake by CD36 polypeptides in various cells, such as macrophages. The invention also relates to the use of such compounds, in particular for research, development, improvement, diagnosis, therapeutic or prophylactic treatment of various pathological conditions. The invention also relates to the products or compositions, such as CD36 variants, recombinant cells, vectors, kits, and the like, which can be used for implementing the above methods or for therapeutic purposes.

CD36 is a highly glycosylated glycoprotein expressed by monocytes, macrophages, platelets, microvasculature endothelial cells, erythrocyte precursors, mammary epithelial cell and adipocytes.¹⁻⁷ This 88 kDa adhesion molecule, member of a family comprising several other genes, was first shown to be receptor for thrombospondin and collagen.^{8.9} It was then also implicated in the phagocytosis of apoptotic neutrophils in tandem with αvβ3.¹⁰⁻¹² The site on CD36 binding to apoptotic neutrophils is localized between amino acids 155 to 183.¹¹ CD36 is also involved in the cytoadherence of erythrocytes infected with *plasmodium Falciparum*.¹³

More recently, CD36 expressed by monocytes/macrophages was shown to play a major role in the endocytosis of OxLDL and fatty acids.^{7,14,15} In our laboratory, we have localized the OxLDL binding site on CD36 to a region encompassing amino acids 155-183.¹⁶ The uptake of OxLDL by macrophages is a crucial step in the initiation and the development of atherosclerotic lesions. Indeed, CD36 plays a major role in the uptake of OxLDL by macrophages and in their differentiation into foam cells within human atherosclerotic lesions.^{17,18} OxLDL also induces an increase of CD36 expression in human monocyte-derived macrophages and in endothelial cells of the microvasculature of mice hearts fed a high-fat diet.^{19,20} The critical role of CD36 in monocytes differentiation, as shown by Tontonoz *et al* and Nagy *et al*, is linked to its capacity for OxLDL uptake. OxLDL taken up by CD36 induces the activation of peroxisome proliferator-activated factor receptor γ (PPAR γ) and the subsequent lipid accumulation in macrophages.^{21,22} Mice with a CD36 deficiency, in a knockout mice model, show a high plasmatic level of cholesterol, nonesterified free fatty acids and triacylglycerol. This knockout mouse model strongly confirms the major role of this adhesion molecule in the binding and uptake of OxLDL.²³ Moreover, a CD36-ApoE double null mice showed the importance of the class B scavenger receptor CD36 in the development of atherosclerotic lesions.⁵⁸ Indeed, the absence of CD36 in this atherogenic animal model (ApoE-null strain) dramatically decreased the size of aortic lesions, inhibited lipid accumulation and foam cell formation.

Hypertension and hypertriglyceridemia, in spontaneously hypertensive rats (SHRs), have also been linked to a lack of CD36 expression and a number of other genes in adipocyte tissues of these animals.²⁴ More recently, a single segment of chromosome 4, from the normotensive Brown Norway rat, was transferred onto the SHR background and it induced a reduction in systolic blood pressure and an amelioration in fructose-induced glucose intolerance, hyperinsulinemia and hypertriglyceridemia.²⁵ In contrast, another study has indicated that the absence of CD36 in the SHR strain is unlikely to be a major cause of insulin-resistance phenotypes.²⁶

Increasing evidence is available to show that CD36 is implicated in intracellular signaling events. Ligands, such as thrombospondin, collagen, or monoclonal antibodies, binding to CD36 can induce platelet activation and superoxide anion production in monocytes.²⁷⁻²⁹ Thrombospondin, in a similar manner to signal transducing multimeric ligands such as tumor necrosis factor or interferon gamma, has the capacity to induce a dimerization of membrane expressed CD36.³⁰⁻³² This ligand-induced receptor dimerization is an important step in events leading to signal transduction. Interestingly, the cytoplasmic tail of CD36 is associated with protein tyrosine kinases of the src family (yes, fyn, lyn) in platelets and in human dermal microvascular endothelial cells.^{33,34} Recently, a study of Lipsky *et al* has shown that the carboxyl-terminal cytoplasmic domain of CD36 is required for OxLDL modulation of NF-KB activity.³⁵ However, the nature of the CD36-protein kinase association and the mechanisms by which CD36 may function in ligand binding and in signal transduction remain to be elucidated.

The present invention stems from an analysis of the functional components of CD36 and now shows, for the first time, a specific site on the CD36 cytoplasmic tail that is critical for the binding, endocytosis and the targeting of OxLDL. The present invention more particularly demonstrates that the cytoplasmic domain of CD36 is important not only for signal transduction but also for oxLDL uptake, and that compounds may be selected that modulate oxLDL uptake through their interaction with CD36 cytoplasmic domain. Accordingly, the present invention now proposes novel methods of screening or identifying compounds that modulate oxLDL cellular uptake, by measuring cellular uptake of labeled oxLDL in the presence or absence of various candidate compounds. More preferably, the compounds are selected based on their ability to inhibit oxLDL cellular uptake through modulation of CD36 cytoplasmic domain. Another aspect of this invention resides in the use of a compound that interacts with the cytoplasmic domain of a CD36 polypeptide for reducing oxLDL uptake by cells, in particular macrophages, in vitro, ex vivo or in vivo. The method is suitable for the treatment of various pathological conditions such as atherosclerosis, for instance. Other aspects of this invention reside in particular CD36 polypeptides having reduced oxLDL uptake ability, corresponding nucleic acid molecules, recombinant cells, and uses thereof.

An object of the present invention more particularly resides in a method of screening or identifying compounds that modulate oxLDL cellular uptake, comprising : (i) separately contacting cells expressing a CD36 polypeptide, in the presence of labelled oxLDL, with one or several candidate compounds, (ii) measuring oxLDL uptake by the cells in the presence of the candidate compounds and (iii) comparing the oxLDL cellular uptake measured in presence of the candidate compounds to the oxLDL cellular uptake measured in absence of candidate compounds to select or identify compounds that cause a modulation of oxLDL cellular uptake.

Within the context of the present invention, oxLDL cellular uptake includes oxLDL binding to a CD36 polypeptide, oxLDL endocytosis (i.e., internalisation) and/or degradation by the cells. In this respect, the method is more particularly suited for screening or identifying compounds that inhibit or reduce the binding and/or endocytosis of oxLDL by cells.

Within the context of the present invention CD36 designates the human wild-type (wt)CD36 polypeptide having the sequence as described in Accession Number L06850 (SEQ ID NO:1). A CD36 polypeptide also includes any variants or homologs of the above human wt sequence, more particularly naturally-occurring variants or homologs, such as polymorphisms, splicing variants, etc., that have the ability to uptake oxLDLs. CD36 polypeptides also include other members of the CD36 gene family such as CD36L1 or CD36L2 polypeptides, which are expressed by hepatocytes. More generally, CD36 polypeptides include any polypeptide encoded by a nucleic acid molecule that hybridises, under stringent condition, with a nucleic acid encoding the human wtCD36 as described above, and having the ability to mediate oxLDL uptake.

As mentioned above, this invention relates to methods of screening or identifying modulators of oxLDL cellular uptake. The present invention is more particularly suited to screen or identify compounds that inhibit or reduce oxLDL cellular uptake. In this respect, inhibition does not imply complete suppression of oxLDL uptake, but also encompasses partial inhibition, in particular a reduction in oxLDL uptake. Preferably, the selected compounds have the ability to reduce by at least 30% the oxLDL uptake obtained in the absence thereof, even more preferably by at least 40%, still preferably by at least 50%.

In a particular embodiment of the method of the present invention the oxLDL cellular uptake measured in presence of the candidate compounds in further compared to the oxLDL cellular uptake by a recombinant cell expressing a CD36 variant lacking a functional cytoplasmic region, in the absence of the candidate compounds. More particularly, as indicated above, the present invention now discloses, for the first time, that the cytoplasmic region of CD36, although not exposed at the cell surface, controls or influences oxLDL uptake, including oxLDL binding and internalisation. In this respect, the present invention unexpectedly shows that CD36 variants (R467STOP and K472STOP) with partial deletions of the C-terminal cytoplasmic tail, exhibit significant decreases in binding and internalization (53.9% and 38.5% respectively) and degradation (68.6% and 51.1% respectively) of OxLDL compared to wild-type CD36. These data indicate, for the first time, a major role for the CD36 C-terminal cytoplasmic tail in OxLDL uptake and provide a very advantageous means of selecting compounds that modulate oxLDL activity (or uptake).

Lack of a functional cytoplasmic domain can result from various modifications, including mutation(s), deletion(s) and/or insertion(s) of one or several amino acid residues within the cytoplasmic domain. More preferably, the cytoplasmic domain of CD36 comprises the following CD36 C-terminal amino acid residues : 462 is Ser Tyr Cys Ala Cys Arg Ser Lys Thr Ile Lys is 472 (amino acids 462-472 of CD36, corresponding to residues 462 to 472 of SEQ ID NO: 1).

In this respect, in a particular embodiment, the method of this invention comprises a comparison of the oxLDL cellular uptake measured in presence of a candidate compound to the oxLDL cellular uptake by a recombinant cell expressing a CD36 variant lacking one or several of the six C-terminal amino acid residues. As specific examples, the CD36 variant may by K472STPOP, lacking the C-terminal lysine amino acid residue or R467STOP, lacking the six C-terminal amino acid residues.

In a more preferred embodiment, the present invention thus resides in a method of screening or identifying compounds that inhibit or reduce oxLDL cellular uptake, the method comprising:
(i) separately contacting recombinant cells expressing a CD36 polypeptide with one or several candidate compounds, in the presence of labelled oxLDL,
(ii) determining oxLDL uptake by said cells,
(iii) comparing said oxLDL uptake to reference uptakes obtained in the absence of a candidate compound (a) by the same recombinant cells and (b) by recombinant cells expressing a CD36 variant lacking a functional cytoplasmic domain.

An other object of the present invention resides in a method of screening or identifying compounds that interact with the cytoplasmic domain of CD36, comprising contacting cells expressing a CD36 polypeptide with one or several candidate compounds in the presence of labelled oxLDL and selecting the compounds which cause a reduction or an inhibition of oxLDL uptake in comparison to the cells in the absence of candidate compounds.

The above methods of the present invention may be performed with various cells or cell populations that express CD36. More preferably, the cells express human wtCD36. The cells may be any mammalian cell naturally expressing CD36, such as macrophages, endothelial cells, erythrocytes, epithelial cells, hepatocytes, etc. Preferably, the cells are recombinant cells, which contain a nucleic acid molecule encoding the CD36 polypeptide. In this respect, recombinant mammalian, lower eukaryotic, or even prokaryotic cells may be employed. Recombinant mammalian cells are preferred, such as human, rodent, hamster, dog, etc. The use of human mammalian cells which do not naturally express CD36 is advantageous since it provides an increased selectivity of the method and still provides a functional assay system for oxLDL uptake. Specific examples of such cells include HEK293 and human Bowes melanoma cells, for instance. It should be understood that the invention is not limited to the choice of a particular cell type. Recombinant cells expressing a CD36 polypeptide may be produced according to conventional molecular biology techniques, by introducing a nucleic acid construct into the cells. Delivery of the nucleic acid may be performed by any known method such as calcium precipitation, electroporation, naked DNA, lipid-mediated transfection, polymer-mediated transfection, peptide-mediated transfection, viral-mediated transfection or infection, etc, as disclosed in the examples. The transformants may be selected using conventional techniques (antibiotic resistance, marker gene, CD36 antibody, etc.).

In the present invention, the cells may be cultured in any appropriate medium, including RPMI, DMEM, AIMV, etc. Usually, between 10⁴ and 10⁷ cells are used for each assay, more preferably between 10⁵ and 10⁶.

In performing the present invention, labelled oxLDL is used in order to detect or measure oxLDL uptake. OxLDL may be labelled according to various techniques, such as radioactivity, fluorescence, luminescence, enzymatic, biological, chemical, etc. In a typical embodiment, the oxLDL is radio-labelled. Radio-labelling may be accomplished using various isotopes such as ¹²⁵I, ³H, ¹⁴C, ³⁵S, ³²S, etc. Preferably, oxLDL is iodinated (e.g., ox¹²⁵I-LDL). In another embodiment, oxLDL is labelled with a fluorochrome, such as for instance rhodamine, etc. The radioactive method (using ox¹²⁵I-LDL) is advantageous, since it allows to quantitatively measure binding, internalisation and degradation of oxLDL.

OxLDL concentration may be adjusted by the skilled person, depending on the cell type, concentration, or assay conditions. Typically, between 1-100 µg/ml oxLDL are used for each assay. The specific activity may also be adjusted by the skilled person.

The measure of oxLDL uptake can be performed according to various techniques, depending on the label used. Preferably, measure is performed after washing the samples to remove free labelled oxLDL. The samples may then be fixed for measuring fluorescence or intracellular labelling. Cell pellets may also be prepared for analysis. In a particular embodiment, measuring oxLDL uptake is performed by determining the amount of labelled oxLDL present in the cell or at the cell surface. Furthermore, it should be noted that oxLDL may be replaced with specific CD36 ligands or agonists.

An other object of this invention relates to a method of screening or identifying compounds that modulate oxLDL uptake, comprising contacting one or several candidate compounds with a polypeptide comprising all or part of the cytoplasmic domain of CD36 and selecting the compounds that interact with said polypeptide, said compounds being candidate modulators of oxLDL uptake. More preferably, the polypeptide comprises at least the sequence of amino acid residues 462-472 of human wtCD36. The polypeptide may be natural (obtained from CD36 extracted from tissue material), recombinant, or synthetic. The polypeptide may comprise the entire CD36 sequence, or a portion thereof. In this regard, the polypeptide may be a peptide, such as a 10 (or less) amino acids long peptide. The polypeptide may further comprise additional sequence, to facilitate anchoring to a support, or synthesis, or purification, for instance.

The present invention can be used to screen various candidate compounds, including any isolated product or any mixture or composition. The compound may be an isolated and structurally-defined product, an isolated product of unknown structure, a mixture of several known and characterized products or an undefined composition comprising one or several products. Examples of such undefined compositions include for instance tissue samples, biological fluids, cell supernatants, vegetal preparations, etc. The test compound may be any organic or inorganic product, including a polypeptide (or a protein or peptide), a nucleic acid, a lipid, a polysaccharide, a chemical product, or any mixture or derivatives thereof. The compounds may be of natural origin, synthetic origin, including libraries of compounds.

The screening methods of the present invention (or at least the contacting step) may be performed in any appropriate device or support, such as plates, dishes, tubes, columns, etc. A preferred embodiment uses a multi-well plate (especially the 96-wells, 384-wells or higher), allowing the separate screening of several candidate compounds to be performed simultaneously. In this regard, in a preferred embodiment, several candidate compounds are separately and simultaneously tested on the cell population. To that effect, samples of the cell population are distributed in separated spots (or wells) of the support (e.g., plate) and each candidate compound is contacted separately with a sample.

The present invention also relates to CD36 polypeptide variants as described above, more particularly to any CD36 polypeptide variant lacking only the C-terminal amino acid residue. More specifically, this invention relates to the particular CD36 polypeptide variant K472STOP, which is a variant of human wtCD36 lacking the C-terminal lysine amino acid residue. As mentioned above, the results presented in this application demonstrate, unexpectedly, that the removal of one single amino acid at the C-terminal end of CD36 significantly decreases the ability of the polypeptide to cause binding, internalisation and degradation of oxLDL. This result is more particularly surprising since the oxLDL binding site has been initially located on residues 155-183 of human wtCD36. This is further surprising since this represents the first evidence that a modification of the C-terminal region of CD36, which is not known to be exposed at the cell surface, and which is distant from the oxLDL binding site, significantly affects oxLDL uptake.

The invention also encompasses any nucleic acid molecule encoding a CD36 polypeptide variant as described above, more particularly the K472STOP variant, as well as any vector comprising such a nucleic acid. The nucleic acid may be any DNA or RNA molecule, such as cDNA, synthetic or semi-synthetic DNA, mRNA, etc. The nucleic acid may be produced according to techniques known in the art, such as by cloning, ligation, enzymatic restriction, artificial synthesis, etc., alone or in combinations. The vector may be any plasmid, episome, virus, phage, artificial chromosome, etc.

The invention further relates to recombinant cells comprising a nucleic acid or a vector as defined above. The cell may be any mammalian primary cell or established cell culture, as well as any lower eukaryotic (e.g.; yeast) or prokaryotic (e.g., bacteria) cell. Preferably, the cell is a mammalian cell, such as a human cell or cell line, or a rodent cell or cell line. It should be understood that the type of cell may be selected by the skilled person depending on the type of vector or nucleic acid, the intended used of the cell, etc. For instance, for screening purposes, as mentioned above, mammalian cells would be preferred. In other instances, prokaryotic or lower eukaryotic cells may be preferred or selected, in particular for producing CD36 variant, or further manipulation of the vector or nucleic acid.

An other object of the present invention resides in the use of any compound obtainable by the above methods for therapeutic, diagnostic, research or development purposes. In this respect, a particular object of this invention resides in the use of a compound that interacts with the cytoplasmic domain of a CD36 polypeptide for the manufacture of a composition for reducing oxLDL uptake by cells, in particular macrophages.

More preferably, the composition is directed at reducing oxLDL binding to macrophages and/or at reducing oxLDL endocytosis by macrophages.

Even more preferably, the compound is a compound that interacts with at least a portion of the C-terminal region of a CD36 polypeptide, even more preferably with at least a portion of the sequence Cys Arg Ser Lys Thr Ile Lys. This sequence represents the last seven C-terminal amino acid residues of human wtCD36 (residues 466 to 472 of SEQ ID NO: 1). As indicated in this application, any alteration of the structure, conformation, binding or, more generally, the functionality of this domain significantly impacts on the oxLDL cellular uptake.

Another object of the present invention resides in the use of a compound that interacts with the cytoplasmic domain of a CD36 polypeptide for the manufacture of a composition for preventing, reducing or treating atheroscerosis. Indeed, oxLDL uptake (including the internalisation and degradation thereof) by macrophages is a crucial step in the initiation and the development of atherosclerotic lesions. Accordingly, by reducing oxLDL uptake in accordance with the present invention, it is possible to reduce or prevent or treat atherosclerosis. As mentioned above, the compound is more preferably a compound that interacts with at least a portion of the C-terminal region of a CD36 polypeptide, even more preferably with at least a portion of the sequence Cys Arg Ser Lys Thr Ile Lys (residues 466 to 472 of SEQ ID NO: 1).

The compound may be of various origin or nature, preferably a synthetic compound such as small chemical entities, peptides, polypeptides, RNAs, PNAs, DNAs, etc.

This invention also relates to kits for implementing the above methods, comprising recombinant cells expressing a CD36 polypeptide, and/or labelled oxLDL. Preferred kits include a recombinant cell expressing a CD36 polypeptide variant lacking the C-terminal amino acid residue, more preferably the K472STOP variant. The kits may further comprise protocols and/or reagents to measure and/or compare labelling.

Other aspects and advantages of the present invention will be disclosed in the following experimental section, which should be regarded as illustrative and not limiting the scope of the present application.

### Legend to the Figures

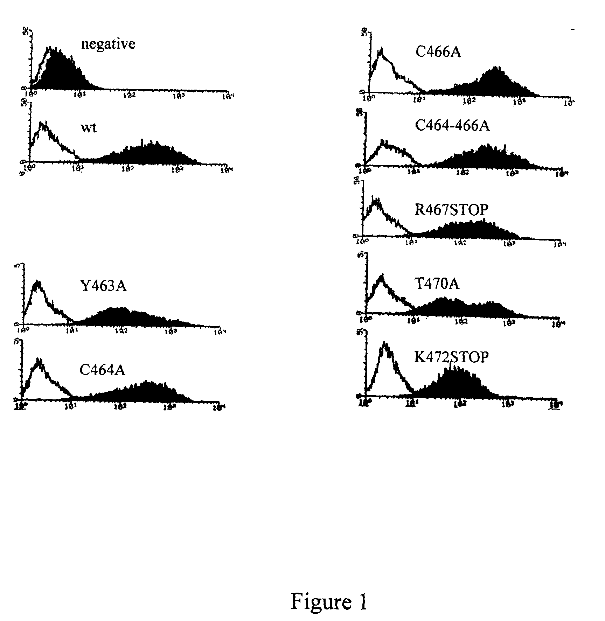

**Figure 1.** Expression of CD36 in human embryonic kidney 293 cells (HEK293). Flow cytometry analysis of CD36-transfected cells and control HEK293 cells in the absence or presence of anti-CD36 antibody 10/5 for each CD36 construct. HEK293 cells were detached, washed, incubated with 5 µg/ml anti-CD36 antibody 10/5 1 h at 4°C, washed, then incubated with anti-mouse FITC antiboby 45 min at 4°C, and resuspended in PBS and fixed with 1% paraforamaldehyde and analysed in FACScan with LysisII software.

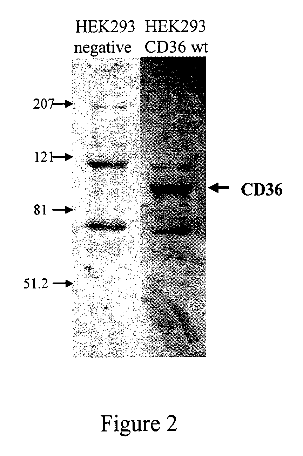

**Figure 2.** Characterization of CD36 stable-transfected HEK 293 cell lines. Western blot analysis of cell lysates from transfected CD36 constructs. Cell lysate proteins were separated by SDS-PAGE (50 µg of protein), transferred to nitrocellulose, probed with 10 µg/ml anti-CD36 antibody 10/5, and detected by chemiluminescence. ; *lane 1*, not-transfected HEK 293 cells ; *lane 2,* CD36-transfected HEK 293 cells.

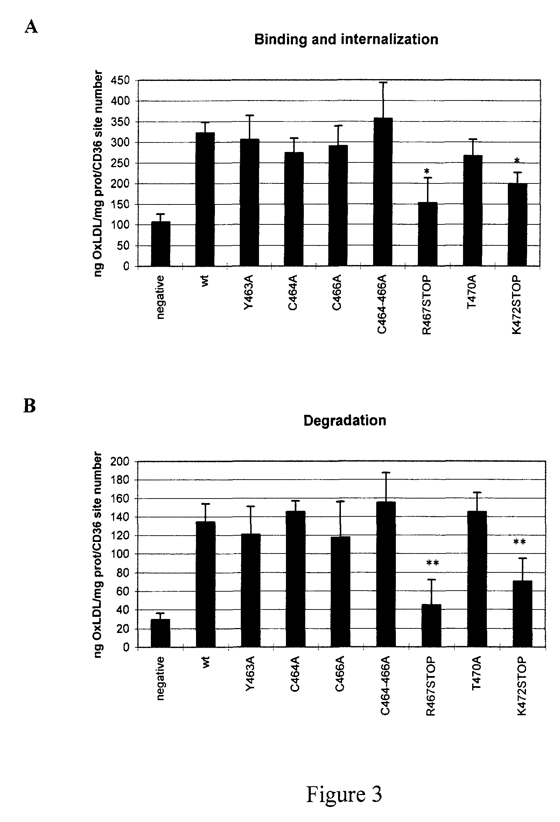

**Figure 3.** Ox¹²⁵I-LDL fixation, internalisation and degradation by stably transfected CD36-HEK293 cell lines. CD36 stable transfection were incubated with 10 µg/ml Ox¹²⁵I-LDL for 4 h at 37°C in the absence or presence of 40-fold excess of Ox-LDL to measure non-specific binding. Values represent the specific binding and internalization (*panel A*) or degradation (*panel B)* of three triplicate wells (mean ± SD). ∗ p< 0.01 compared with human wild-type CD36. ∗∗ p< 0.05 compared with human wild-type CD36.

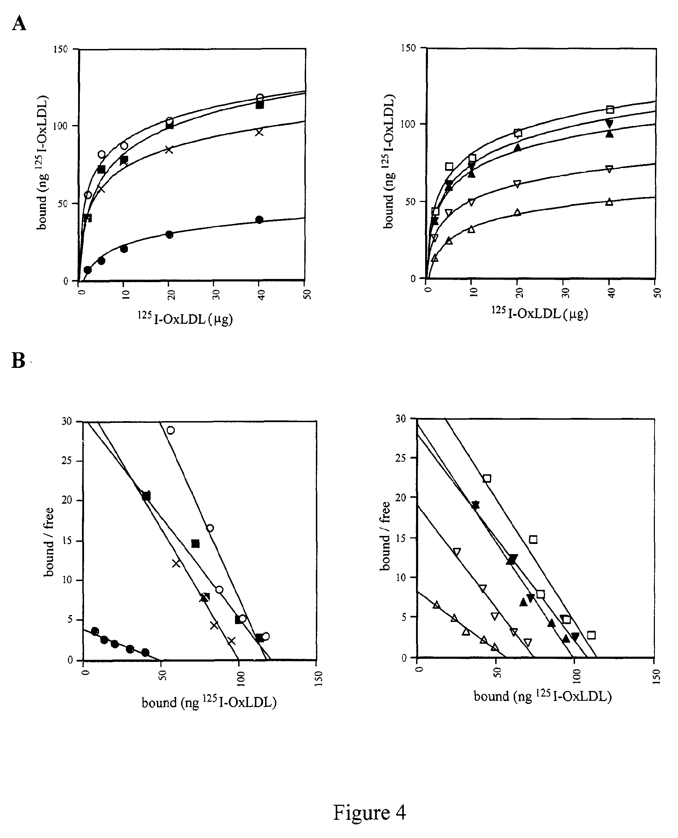

**Figure 4.** Binding of Ox¹²⁵I-LDL to stably transfected CD36-HEK293 cell lines. Cells were incubated with Ox¹²⁵I-LDL at different concentrations (2, 5, 10, 20 and 40 µg/ml) for 1 h at 4°C in the absence or presence of 15-fold excess of Ox-LDL. Cells was washed three times in PBS, 0.5% BSA and three times in PBS, then bound radioactivity was counted after solubilizing the well contents in 0.1 N NaOH. Each point represents the mean of three experiments. *Panel A-* Binding of Ox¹²⁵I-LDL to CD36 constructs. *Panel B-* Scatchard analysis calculated using linear regression. ● negative; ○ wt; x Y463A; ■ C464A; □ C466A; ▲ C464-466A; Δ R467STOP; ▼ T470A and V K472STOP.

### Materials and Methods

### 1.1. Cell culture

Human epithelial kidney 293 cells (HEK293) were maintained in Minimal Essential Medium (Life technologies) supplemented with 10% fetal bovine serum, 2 mmol/L L-Glutamine, 10 µg/ml streptomycin and 10 units/ml penicillin in 5% CO₂ at 37°C.

### 1.2. Isolation, Modification and Labeling of OxLDL

LDL were isolated from normolipidemic human plasma (Transfusion Center, Rungis, France) using a single-step discontinuous gradient in a Beckman NVT65 rotor.³⁶ Protein content was determined using the BCA assay kit (Pierce). Prior to chemical modification, LDL were dialyzed against 0.01 mol/L phosphate-buffered saline (pH 7.4). Copper-oxidized LDL were prepared under sterile conditions by incubating 500 µg/ml of LDL with 2.5 µmol/L CuCl₂ for 48 h at 37°C. At the end of the incubation period, OxLDL were extensively dialyzed at 4°C against PBS (pH 7.4). OxLDL were characterized by measuring thiobarbituric acid reactive substances (TBARS : 40 ± 5 nmol equiv. MDA/mg LDL protein) and lipoperoxides (LPO : 259 ± 90 nmol/mg LDL protein).^{37,38} Labeling of OxLDL with iodine 125 was prepared using the Iodo-gen reagent (Pierce). Specific activity, determined for each experiment, ranged from 200 to 400 cpm/ng OxLDL. OxLDL was also labeled with rhodamine succinimidyl ester (Molecular Probes) as described Stanton *et al.*³⁹ All samples were filtered through 0.4 µm filters.

### 1.3. DNA Constructs and Transfection

Human wt CD36 cDNA was cloned into the HindIII and XbaI sites of M13mp19. Alanine substitutions or stop codons were introduced into the cDNA sequence by oligonucleotide-directed mutagenesis.⁴⁰ In each case, the codon degeneracy was exploited to introduce a novel restriction-endonuclease site to allow a rapid identification of mutants without altering the coding sequence.⁴¹ The ligation products were amplified in *Echerichia coli* MC1061p3 and purified by a CsCl gradient. All mutations were confirmed by DNA sequencing. CD36 cDNA constructs were cloned into the XbaI sites of a stable expression vector, pCDNA3.1. Each CD36 cDNA construct is represented in Table I. DNA transfection was performed in HEK293 cells using Lipofectamine (Life Technologies) for 2 h at 37°C. The cells were selected 48 h after transfection in medium containing 500 µg/ml G418. Each clone was purified by anti-CD36 monoclonal antibody (10/5) coupled with magnetic beads (Dynal SA).

### 1.4. Western Blotting

Confluent cells, in a 25 cm² flask were detached by Trypsin/EDTA, washed twice phosphate-buffered saline (PBS), pH 7.4, then lysed into 200 µl of 0.02 mol/L TRIS, 0.15 mol/L NaCl containing anti-proteases and 1% lubrol and rocked for 25 min at 4°C. Lysates was then purified by centrifugation. Cell lysates were separated by 10% SDS-polyacrylamide gel electrophoresis (SDS-PAGE). Proteins were transferred to a nitro-cellulose membrane and blocked for 4 h at room temperature in blocking buffer (Tris-buffered saline (TBS) pH 7.6, 0.05% Tween 20 and 5% nonfat dry milk).After a night incubation at 4°C, with primary antibody (10/5 at 10 µg/ml), the blot was incubated with appropriate secondary anti-IgG-horseadish peroxidase conjugate. The membrane was washed three times for 30 min in TBS,0.05% Tween 20, and developed with ECL chemiluninescent substrate (Amersham).

### 1.5. Flow Cytometry

1.10⁶ cells/ml were washed twice with phosphate-buffered saline (PBS, pH 7.4), then incubated with the anti-CD36 monoclonal antibodies (mAbs 10/5 or 13/10) for 1 h at 4°C (10 µg/ml) in PBS, 3% BSA. These mAbs 10/5 and 13/10 directed against CD36 were produced and characterized in our laboratory.⁴² After one wash in PBS, cells were incubated with goat anti-mouse IgG fluorescein isothiocyanate conjugate (DAKO) for 45 min at 4°C. Cells were washed once in PBS then fixed with 1% formaldehyde. The expression level of CD36 and its variants was assessed by flow cytometry using Becton-Dickinson Facscan and LysisII software. The CD36 sites number of each stable transfected cell line was also quantified with fluorescent microbeads using Dako Qifikit (DAKO).

### 1.6. Assay for OxLDL Rhodamine Endocytosis

2.10⁵ cell of each stable transfected cell line were placed on glass coverslips in 35 mm Petri dishes. After 72 h, cells were incubated with 10 µg/ml rhodamine-OxLDL for 30 min at 37°C. After three washes in phosphate-buffered saline (PBS), cells were fixed with 3% paraformaldehyde for 20 min. After two further washes in PBS, cells were mounted in fluorescent mounting medium (DAKO).

### 1.7. Binding, internalization and Degradation of OxLDL

2.10⁵ cells of each stable transfected cell line were placed in 35 mm Petri dishes for 72 h.
Binding of OxLDL: Cells were incubated with different concentrations of 0x¹²⁵I-LDL (2, 5, 10, 20 and 40 µg/ml) in the presence or absence of 15-fold excess of unlabeled OxLDL for 1 h at 4°C. Cells were then washed twice with PBS 0.5 mmol/L CaCl₂, 0.5 mmol/L MgCl₂ containing 0.2% BSA and twice with PBS 0.5 mmol/L CaCl₂, 0.5 mmol/L MgCl₂. Washed cells were then dissolved in 0.1 N NaOH and scraped off from the plates. An aliquot was used to assay for radioactivity and protein content.
Binding, uptake and degradation of OxLDL: Cells were incubated with 10 µg/ml Ox¹²⁵I-LDL in presence or absence of 40-fold excess of unlabeled OxLDL for 4 h at 37°C. After these incubations at 37°C, OxLDL proteolytic degradation was measured as trichloroacetic acid- and silver nitrate-soluble radioactivity released into the medium. Cells were washed twice with PBS 0.5 mmol/L CaCl₂, 0.5 mmol/L MgCl₂ containing 0.2% BSA and twice with PBS 0.5 mmol/L CaCl₂, 0.5 mmol/L. Washed cells were then dissolved in 0.1 N NaOH and scraped off from the plates. OxLDL cell association was measured as radioactivity contained in the washed and solubilized cell pellets. Protein concentrations was also measured using an aliquot of solubilized cell pellets as described below.

### 1.8. Protein Measurement

Protein concentrations were determined by the method of Lowry *et al* with BSA as the standard using Micro Protein Determination kit (Sigma).⁴³

### 1.9. Statistics

Data are presented as mean±SD. Differences were analyzed by student's t test. A level of P<0.05 was accepted as statistically significant.

### Results

### 2.1. Stable Expression of CD36 in HEK293 Cells

Human epithelial kidney cells were transfected with a stable expression vector bearing different CD36 constructs, having either punctual mutations or deletions, localized in the carboxyl-terminal cytoplasmic domain of CD36 (Table 1). Surface expression of the different CD36 constructs in HEK293 was assessed by flow cytometry using anti-CD36 monoclonal antibodies 10/5 (Figure 1) and 13/10 (results not shown). Monoclonal antibody 10/5 recognized the different CD36 transfected, but not the non-transfected, HEK293 cells. A heterogeneous expression between the different transfected cell lines was observed. Quantification of surface CD36 expression, for comparison between different cell constructs and ensuing endocytosis, was performed using Dako Qifikit (results not shown). Stable, but not non-transfected, HEK293 cells showed by Western blot a band of 88 kDa which represents the protein of the CD36 wild type construct (Figure 2). The 7 other constructs of CD36 have been also characterized by Western blot and they also showed a band, similar to the wild type, recognized by monoclonal antibody 10/5 (data not shown).

### 2.2. Binding and endocytosis of Rhodamine-OxLDL

OxLDL labeled with rhodamine, bound and endocytosed via the different CD36 constructs expressed by HEK293 cells, was localized at the cellular membrane level and in cytoplasmic vesicles. The extent of binding and internalization of rhodamine-OxLDL was dependent upon the type of construct. Constructs R467STOP and K472STOP, unlike the wild type or other constructs, showed decreased binding and internalization of OxLDL (data not shown).

### 2.3. Binding, Internalization and Degradation of Ox¹²⁵I-LDL

¹²⁵I-labeled OxLDL were used for binding, internalization and degradation studies to characterize the different CD36 constructs expressed by HEK293 cells. Two constructs (R467STOP and K472STOP), with partial deletions of the C-terminal cytoplasmic tail, showed significant decreases in binding and internalization (53.9% and 38.5% respectively; p<0.01) and degradation (68.6% and 51.1% respectively; p<0.05) of Ox¹²⁵I-LDL (10 µg/ml) compared to wild-type CD36 (Figure 3A and Figure 3B). These data suggest a major role for the CD36 C-terminal cytoplasmic tail in OxLDL endocytosis. Several other punctual constructs were performed on the cytoplasmic tail. Constructs mutating cysteine to alanine (C464A, C466A and C464-466A) did not affect the endocytosis of OxLDL (Figure 3A and Figure 3B). This would tend to suggest that the conformation of the C-terminal cytoplasmic tail is not required for OxLDL endocytosis. Moreover, two further punctual constructs of amino acids that could possibly be implicated in signal transduction (Y463A and T470A) did not affect OxLDL uptake and degradation (Figure 3A and Figure 3B).

### 2.4. Ox¹²⁵I-LDL affinity to CD36 constructs

Scatchard analysis was used to investigate the affinity of OxLDL to the wild-type and the different CD36 constructs. Increasing concentrations of Ox¹²⁵I-LDL (2, 5, 10 ,20 and 40 µg/ml) were added to the wild-type and to different CD36 constructs expressed by HEK293 cells. Constructs, with the exception of R467STOP, and wild type did not show a difference in the binding of OxLDL (n=3). Binding of OxLDL to R467STOP was lower than that observed for the wild-type and other constructs (Figure 4A). The association constant (*Ka*) of the different CD36 constructs, after correction for the contribution of non-transfected HEK293 cells, was obtained via Scatchard analysis (Figure 4B and Table 2). These results represent three separate experiments with three different preparations of OxLDL. *Ka* for all constructs, except R467STOP, showed no significant difference (Table 2).

### Discussion

This invention identifies for the first time specific sites on the CD36 cytoplasmic tail that are involved in the uptake (e.g., binding, internalization and/or degradation) of OxLDL. Several lines of evidence clearly implicate specific sites of the CD36 cytoplasmic tail in this crucial scavenging activity. 1- Mutant K472STOP significantly reduces, compared to wild-type, the binding, internalization and degradation of OxLDL. 2- Indeed, CD36 functions are considerably affected by changes brought about to a CXCX₅K motif, induced by a deletion in the last amino-acid as shown in construct K472STOP. 3- Construct R467STOP, where the last six amino-acids were truncated, shows significantly reduced OxLDL binding. 4- However, K472STOP in line with the wild type, but in contrast to R467STOP, does not show a decrease in OxLDL affinity. 5- Changes induced in the conformation of the C-terminal cytoplasmic tail, as modulated by punctual mutation at C464A, C466A and C464-466A, did not affect the normal binding and internalization of OxLDL.

CD36 has been shown to bear palmitoylated N- and C-terminal cytoplasmic tails.⁴⁴ The presence of palmitoylated cysteine residues strongly suggests the presence of two intracellular domains. Moreover, assignment of disulfide bridges in bovine CD36 suggests that the bovine CD36 contains two short (residues 1-6 and 461-471) intracellular and transmembrane domains.⁴⁵ In contrast, two other results offer the possibility of only one cytoplasmic domain located at the C-terminal end of CD36.^{29,46} In these last results, authors have truncated the CD36 protein, by removing the intracytoplasmic and transmembrane domains (deletion of 43 amino acids), and showed that this resulted in a secreted form of CD36.

The present application discloses the production of two CD36 mutants (constructs R467STOP and K472STOP), bearing truncated C-terminal cytoplasmic tails, that very significantly affect CD36 functions. Our results argue for a determining role of the C-terminal tail in the control of CD36 functions. Moreover, such results complement those obtained by Lipsky *et al* who show that a domain at the C-terminal end of CD36, the last 6 amino acids, is implicated in the activation of NF-κB following TNFα and OxLDL activation.³² However, Lipsky et al discloses that their cytoplasmic CD36 mutants retain the ability to bind and internalize oxLDL, in contrast with the present invention.

A significant advance in the elucidation of the mechanism by which CD36-mediates signal transduction was reported by Huang *et al* and Bull *et al.*^{30,31} In these studies, the authors demonstrated that the cytoplasmic tail of CD36 is associated with pp60^{fyn}, pp62^{yes} and pp54/58^{lyn} protein tyrosine kinases of the src gene family in platelets, human C32 melanoma, HEL erythroleukemia and human dermal microvascular endothelial cells. However, the nature of the association between the cytoplasmic protein tyrosine kinases and CD36 has yet to be clearly defined. The C-terminal cytoplasmic tail of CD36 contains a motif (CXCX₅K). Such a motif (CXCX₅K) has been proposed to be implicated in the association between CD4 and tyrosine kinase p56^{lck}87.⁴⁷ Deletion of lysine 472, the last amino acid in the CXCX₅K motif, considerably reduces the functional activity of CD36. A similar effect is observed when arginine 467 and the next 5 amino acids, presented as X₅K in CXCX₅K, are deleted. In contrast, punctual mutations of cysteines to alanine, in the motif, have no effect. The capacity of the motif to control CD36 functions seems to be impaired when the last amino acids are deleted.

Monoclonal antibody or ligand-induced receptor oligomerization is an important step in the signal transduction pathway. Studies have shown that CD36 can exist in a dimer form following ligand binding.^{29,48} Indeed, tumor necrosis factor and interferon-γ induce an aggregation of their receptors and subsequently are involved in signal transduction.^{27,28} F(ab')2 fragments of a CD36 monoclonal antibody (OKM5) activate platelets and induce in monocytes an oxidative burst and the generation of superoxide anion.^{25,26} OxLDL binding to CD36 is implicated in the activation of transcriptional factors such as NF-κB factor and the nuclear receptor PPARγ.^{21,32} Such an activation, hypothetically implicating clustering of CD36 by OxLDL, allows differentiation and an increased expression of CD36 synthesis on monocytes/macrophages. Tontonoz *et al* also show an increase in CD36 expression mediated by the nuclear hormone receptor heterodimer, PPARγ:RXRα.²¹

Induction or reduction, of CD36 expression appears to be controlled by a number of factors. CD36 expression is increased in platelets following thrombin activation and in monocytes in response to several cytokines, and macrophage colony stimulating factor.^{17,49,50} OxLDL, the lipoproteins strongly implicated in the development of atherosclerosis, induce an increase in CD36 expression in human-derived macrophages.¹⁹ Such OxLDL are involved in the mechanisms leading to the transformation of macrophages to foam cells and the modulation of CD36 expression.¹⁷ Moreover, the hearts of diabetic mice show upregulation of CD36 following high-fat diet feeding.²⁰ In contrast, lovastatin, a member of the 3-hydroxy-3methylglutaryl-coenzyme A reductase inhibitors family, induces a reduction of CD36 expression in human monocyte-derived macrophages.⁵¹ Such a drug is widely used in the treatment of hypercholesterolemia. In addition, n-3 fatty acids can induce a down-regulation of CD36 in human monocytic cells.⁵² Finally, interferon-γ, a cytokine produced by activated lymphocytes in atheroma plaques, suppresses CD36 expression in human monocyte-derived macrophages.¹⁹ In the processing of atherosclerosis, monocytes-macrophages are the cells that play an important role in foam cell formation by uptake of OxLDL.⁵³⁻⁵⁷ Recently, the class B scavenger receptor CD36 was shown to be directly implicated in the development of atherosclerotic lesion in a CD36-ApoE double null mice.⁵⁸ The absence of CD36 in this atherogenic animal model (ApoE-null strain) decreased the size of aortic lesions and inhibited lipid accumulation and foam cell formation, further supporting the role of CD36 in atherosclerotic lesion development in vivo. Thus, the modulation of CD36 expression and functions according to this invention is instrumental in controlling the initiation, perpetuation and progression of vascular lesions. This invention shows that a specific site in the C-terminal cytoplasmic domain of CD36, is crucial for the binding, internalization and degradation of OxLDL. This invention also allows a better understanding of the role of CD36 in the mechanism controlling OxLDL binding and endocytosis and provides novel methods to prevent the progression and the development of atherosclerosis in human arterial walls.

### References

1. Silverstein et al., *J Clin Invest.* 1989;84:546-552.
2. Talle et al., *Cell Immunol.* 1983;78:83-99.
3. Tandon et al., *J Biol Chem.* 1989;264:7570-7575.
4. Knowles et al., *J Immunol.* 1984;132:2170-2173.
5. Edelman et al., *Blood.* 1986;67:56-63.
6. Greenwalt et al., *Biochemistry.* 1990;29:7054-7059.
7. Abumrad et al., *J Biol Chem.* 1993;268:17665-17668.
8. Asch et al., *J Clin Invest.* 1987;79:1054-1061.
9. Tandon et al., *J Biol Chem.* 1989;264:7576-7583.
10. Ren et al., *J Exp Med.* 1995;181:1857-1862.
11. Navazo et al., *J Biol Chem.* 1996;271:15381-15385.
12. Savill et al., *J Clin Invest.* 1992;90:1513-1522.
13. Oquendo et al., *Cell.* 1989;58:95-101.
14. Endemann et al., *J Biol Chem.* 1993;268:11811-11816.
15. Nicholson et al., *Arterioscler Thromb Vasc Biol.* 1995;15:269-275.
16. Puente Navazo et al., *Arterioscler Throm. Vasc Biol.* 1996;16:1033-1039.
17. Huh et al., *Blood.* 1996;87:2020-2028.
18. Nakata et al., *Arterioscler Thromb Vasc Biol.* 1999;19:1333-1339.
19. Nakagawa et al., *Arterioscler Thromb Vasc Biol.* 1998;18:1350-1357.
20. Greenwalt et al., *J Clin Invest.* 1995;96:1382-1388.
21. Tontonoz et al., *Cell.* 1998;93:241-252.
22. Nagy et al., *Cell.* 1998;93:229-240.
23. Febbraio et al., *J Biol Chem.* 1999;274:19055-19062.
24. Aitman et al., *Nat Genet.* 1999;21:76-83.
25. Pravenec et al., *J Clin Invest.* 1999;103:1651-1657.
26. Gotoda et al., *Nat Genet.* 1999;22:226-228.
27. Ockenhouse et al., *J Clin Invest.* 1989;84:468-475.
28. Aiken et al., *Blood.* 1990;76:2501-2509.
29. Schuepp et al., *Biochem Biophys Res Commun.* 1991;175:263-270.
30. Greenlund et al., *J Biol Chem.* 1993;268:18103-18110.
31. Pennica et al., *Biochemistry.* 1993;323:3131-3138.
32. Daviet et al., *Thromb Haemost.* 1997;78:897-901.
33. Huang et al., *Proc Natl Acad Sci USA.* 1991;88:7844-7848.
34. Bull et al., *FEBS Lett.* 1994;351:41-44.
35. Lipsky et al., *Recept Signal Transduct.* 1997;7:1-11.
36. Gieseg et al., *FEBS Letters* 1994;343:188-194.
37. Buege et al., *Methods Enzymol.* 1978;52:302-310.
38. el-Saadani et al., *J Lipid Res.* 1989;30:627-630.
39. Stanton et al., *J Biol Chem.* 1992;267:22446-22451.
40. Kunkel et al., *Methods Enzymol.* 1987;154:367-382.
41. McClelland et al., *Proc Natl Acad Sci USA* 1991;88:7993-7997.
42. Daviet et al., *Eur J Biochem* 1997;243:344-349.
43. Lowry et al., *J Biol Chem.* 1951;193:265-275.
44. Tao et al., *J Biol Chem.* 1996;271:22315-22320.
45. Rasmussen et al., *Eur J Biochem* 1998;257:488-494.
46. Frieda et al., *Blood* 1994;84:384-389.
47. Turner et al., *Cell.* 1990;60:755-765.
48. Ibrahimi et al., *Proc Nati Acad Sci USA* 1996;93:2646-2651.
49. Michelson et al., *Arterioscler Thromb* 1994;14:1193-1201.
50. Yesner et al., *Arterioscler Thromb Vasc Biol.* 1996;16:1019-1025.
51. Hrboticky et al., *Arterioscler Thromb Vasc Biol.* 1999;19:1267-1275.
52. Pietsch et al., *Cell Biochem Funct.* 1995;13:211-216.
53. Jonasson et al., *Artheriosclerosis.* 1986;6:131-138.
54. Ross et al., *Nature* 1993;362:801-809.
55. Rosenfeld et al., *Artheriosclerosis.* 1990;10:680-687.
56. Katsuda et al., *Am J Pathol.* 1993;142:1787-1793.
57. DiCorleto et al., *Am J Hypertens.* 1993;6:314S-318S.
58. Febbraio et al., J Clin Invest. 2000 ;105 :1049-1056.

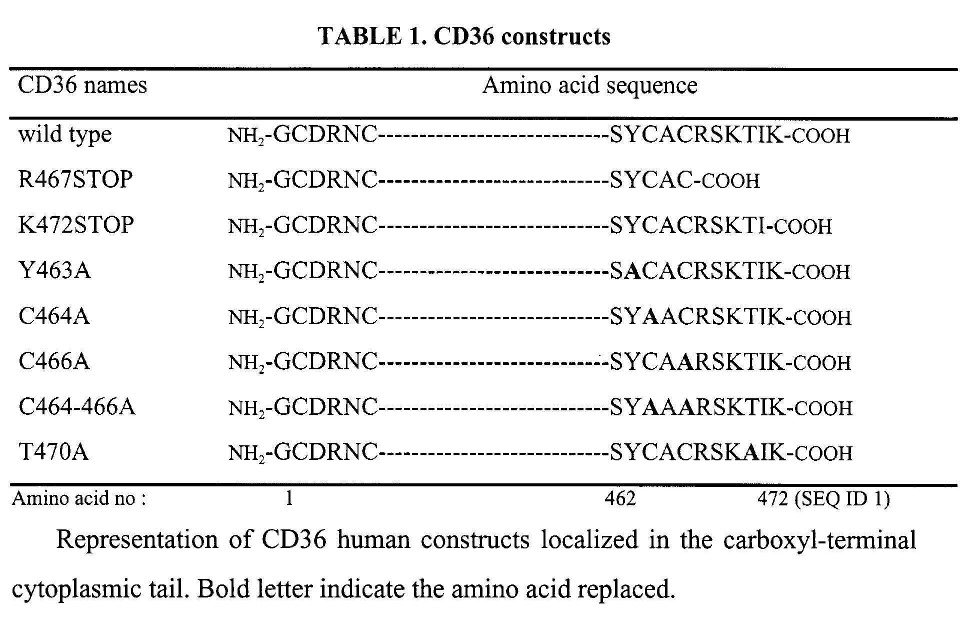

**TABLE 2.**

| **Association constant values of OxLDL for CD36 constructs obtained through Scatchard diagram.** | |
|---|---|
| CD36 names | association constant (*Ka* ± SD 10⁸ L/mol) |
| wild type | 20.67 ± 6.66 |
| R467STOP | 7.45 ± 2.85∗ |
| K472STOP | 13.05 ± 3.15 |
| Y463A | 17.59 ± 5.35 |
| C464A | 13.12 ± 5.37 |
| C466A | 15.7 ± 3.42 |
| C464-466A | 18.11 ± 7.98 |
| T470A | 13.63 ± 4.01 |
| • p=0.03 *Ka* value of R467STOP is significantly different compared with the *Ka* value of wild-type CD36 | |

## Claims

1. A method of screening or identifying compounds that modulate oxLDL cellular uptake, comprising : (i) separately contacting cells expressing a CD36 polypeptide, in the presence of labelled oxLDL, with one or several candidate compounds, (ii) measuring oxLDL uptake by the cells in the presence of the candidate compounds and (iii) comparing the oxLDL cellular uptake measured in presence of the candidate compounds to the oxLDL cellular uptake measured in absence of candidate compounds to select or identify compounds that cause a modulation of oxLDL cellular uptake.

2. The method of claim 1, to screen or identify compounds that inhibit or reduce oxLDL cellular uptake

3. The method of claim 2, to screen or identify compounds that inhibit or reduce the binding or endocytosis of oxLDL by cells.

4. The method of any one of the preceding claims, wherein the cell expressing a CD36 polypeptide is a recombinant cell expressing wild-type human CD36.

5. The method of any one of the preceding claims, wherein the labelled oxLDL is radio-labelled.

6. The method of any one of the preceding claims, wherein measuring oxLDL uptake is performed by determining the amount of labelled oxLDL present in the cell or at the cell surface.

7. The method of any one of the preceding claims, wherein the oxLDL cellular uptake measured in presence of the candidate compounds in further compared to the oxLDL cellular uptake by a recombinant cell expressing a CD36 polypeptide lacking a functional cytoplasmic region, in the absence of the candidate compounds.

8. The method of claim 7, wherein the CD36 polypeptide lacks the C-terminal lysine amino acid residue.

9. The method of any one of the preceding claims, wherein compounds of a library are assayed separately.

10. The method of any one of the preceding claims, to screen or identify compounds that interact with the cytoplasmic domain of a CD36 polypeptide.

11. A method of screening or identifying compounds that inhibit or reduce oxLDL cellular uptake, comprising:
(i) separately contacting recombinant cells expressing a CD36 polypeptide with one or several candidate compounds, in the presence of labelled oxLDL,
(ii) determining oxLDL uptake by said cells,
(iii) comparing said oxLDL uptake to reference uptakes obtained in the absence of a candidate compound (a) by the same recombinant cells and (b) by recombinant cells expressing a CD36 variant lacking a functional cytoplasmic domain.

12. A method of screening or identifying compounds that modulate oxLDL uptake, comprising contacting one or several candidate compounds with a polypeptide comprising all or part of the cytoplasmic domain of CD36 and selecting the compounds that interact with said polypeptide, said compounds being candidate modulators of oxLDL uptake.

13. The method of any one of the preceding claims, wherein the contacting is performed in a multi-well plate.

14. The use of a compound that interacts with the cytoplasmic domain of a CD36 polypeptide for the manufacture of a composition for reducing oxLDL uptake by cells, in particular macrophages.

15. The use according to claim 14, for the manufacture of a composition for reducing oxLDL binding to macrophages.

16. The use according to claim 14, for the manufacture of a composition for reducing oxLDL endocytosis by macrophages.

17. The use according to any one of claims 14 to 16, wherein the compound interacts with at least a portion of the C-terminal region of a CD36 polypeptide.

18. The use according to claim 17, wherein the compound interacts with at least a portion of the sequence CysArgSerLysThrIleLys (residues 466-472 of SEQ ID NO:1).

19. The use of a compound that interacts with the cytoplasmic domain of a CD36 polypeptide for the manufacture of a composition for preventing, reducing or treating atheroscerosis.

20. The use according to claim 19, wherein the compound interacts with at least a portion of the sequence CysArgSerLysThrIleLys (residues 466-472 of SEQ ID NO:1).

21. A CD36 polypeptide variant, wherein the polypeptide lacks only the C-terminal amino acid residue, in particular the K472STOP polypeptide.

22. A nucleic acid encoding a CD36 polypeptide of claim 21.

23. A vector comprising a nucleic acid of claim 22.

24. A recombinant cell comprising a nucleic acid of claim 22 or a vector of claim 23.
